Europäisches Patentamt

European Patent Office

Office européen des brevets

(11) Veröffentlichungsnummer: **0 268 114**
A2

## (12) EUROPÄISCHE PATENTANMELDUNG

(21) Anmeldenummer: 87115808.5

(22) Anmeldetag: 28.10.87

(51) Int. Cl.4: **A61K 7/46** , A45D 34/02

(30) Priorität: 03.11.86 DE 3637319
29.11.86 DE 3640917
22.10.87 DE 3735704

(43) Veröffentlichungstag der Anmeldung:
**25.05.88 Patentblatt 88/21**

(84) Benannte Vertragsstaaten:
**AT BE CH ES FR GB GR IT LI LU NL SE**

(71) Anmelder: **Zernisch KG
Inselstrasse 24
D-4000 Düsseldorf(DE)**

(72) Erfinder: **Zernisch, Peter
Cecilienallee 62
D-4000 Düsseldorf 30(DE)**

(74) Vertreter: **König, Reimar, Dr.-Ing. et al
Patentanwälte Dr.-Ing. Reimar König
Dipl.-Ing. Klaus Bergen Wilhelm-Tell-Strasse
14 Postfach 260162
D-4000 Düsseldorf 1(DE)**

(54) **Riechstoffkomposition und -spender.**

(57) Bei einer Riechstoffkomposition befindet sich in den Poren eines Trägerstoffs ein Riechstoff, dessen Fondnote, beispielsweise ein Nikotinderivat, gegenüber den anderen Riechstoffkomponenten das höchste Wirkungsquantum besitzt und daher die Duftnote prägt. Die Riechstoffkompositionignet sich für einen Riechstoffspender, bei dem im Vereich einer Heizvorrichtung (36) ein Riechstoffhalter und/oder ein Riechstoffdosierer (53, 54, 55) angeordnet ist. Der Riechstoff (41) ist in einem Riechstoffbehälter 37 angeordnet, der mit einem Entsorgungsbehälter (39) und einer Riechstoffführung (38) eine Austauscheinheit bildet.

FIG. 5

EP 0 268 114 A2

## "Riechstoffkomposition und -spender"

Die Erfindung bezieht sich auf eine Reichstoffkomposition und einen Riechstoffspender mit mindestens einem verschließbaren Durchlaß, durch den der Riechstoff nach außen gelangt.

Ätherische Öle etc. werden in flüssiger Form in Flaschen gehandelt. Die Flaschen kann der Verraucher nicht jederzeit und überall bei sich tragen und benutzen. Sie sind zerbrechlich, die Flüssigkeit kann auslaufen, Entnahme und Anwendung sind umständlich und riskant, vor allem durch Überdosierung. Das Hauptproblem is tjedoch die Anwendung: sie geschieht entweder bequem und dabei riskant oder risikolos und dabei unbequem.

Die orale und die perkutane Anwendung ist bequem, aber infolge der hohen Aggressivität der konzentrierten Stoffe riskant, die naturgemäße olfaktorische Aufnahme (Inhalation) ist risikolos aber unbequem: Die den Flaschen häufig beigepackten Inhalationsgläser und Inhalationsmasken können nur zu Hause benutzt werden.

Folglich werden bislang ätherische Öle etc. nur unregelmäßig, selten und unter großem Zeitaufwand zu Hause und in Bädern inhaliert. Mangels ausreichender und gleichmäßiger dosierung bleibt die gewünschte Wirkung aus. Auch besteht die Gefahr einer Überdosierung, denn pflanzliche Stoffe ohne jede Toxizität, zumindest bei starker Dosierung, gibt es nicht.

Je nach der Beschaffenheit des Riechstoffs kann die je Zeiteinheit freigesetzte Riechstoffmenge sehr unterschiedlich sein. Hinzu kommt, daß die Wirkung mit zunehmendem Alter der Riechstoffkomposition immer mehr abklingt, da sich der Riechstoff von der Oberfläche des Trägers her verbraucht und demzufolge die Diffusionswege zur Trägeroberfläche immer länger werden. Des weiteren ist es bei den bekannten Riechstoffbehältern schwierig, die Geruchsintensität zu steuern.

Riech-bzw. Duftstoffe, die sich bei Raumtemperatur ohne äußere Wärmezufuhr nicht im gewünschten Maße verflüchtigen, erfordern eine Heizvorrichtung zur Erzeugung der für das Verdampfen des Riechstoffs erforderlichen Wärme. Dazu eignen sich selbstbeheizte Riechstoffbehälter, die jedoch eine dosierte Wärmemenge liefern und einen Behälter für den gasförmigen, flüssigen, pastösen, körnigen oder stückigen Riechstoff aufweisen müssen. Dabei ergibt sich jedoch das Problem, daß Riechstoffe je nach ihrer Konzentration bzw. Menge häufig unverträglich sind und daher gegen Fehlgebrauch geschützt und/oder auch entsorgt werden müssen. Eine entsprechende vorsorge empfiehlt sich auch beim zum Schnüffeln bestimmten trägergebundenen Riechstoffen insbesondere, wenn sich diese in nachfüllbaren Behältern befinden.

Herkömmliche Riechstoffspender sind im allgemeinen für eine Dauerabgabe des Riechstoffs über einen längeren Zeitraum bestimmt. Ein weiteres Problem stellt daher die Bemessung der bei einer kurzfristigen bzw. spontanen Benutzung erforderlichen Riechstoffmenge dar, da diese dem jeweiligen Wärmeangebot entsprechen muß, um einerseits genügend Riechstoff zu verdampfen und andererseits ein rückstandsarmes Verdampfen mit hoher Ausbeute zu gewährleisten.

Je nach Menge und Beschaffenheit des Verdampfungsrückstandes stellt sich bei selbstbeheizten Riechstoffspendern das Problem einer jeden Fehlgebrauch ausschließenden, darüber hinaus aber auch benutzerfreundlichen Entsorgung des Verdampfungsrückstandes.

Der Erfindung liegt die Aufgabe zugrunde, ätherische Öle, Essenzen und Pflanzenextrakte als Mittel der Aromatherapie im Alltage jederzeit und überall in bestimmter Dosierung ohne großen Aufwand verfügbar zu machen. Der Erfindung liegt des weiteren die Aufgabe zugrunde, einen Riechstoffspender zu schaffen, der einen Fehlgebrauch des Riechstoffs und etwaiger Reststoffe zumindest außerordentlich erschwert und gleichzeitig einer Heizvorrichtung für eine diskontinuierliche Benutzung eine bedarfsgerechte und entsorgungsfreundliche Riechstoffmenge zuführt. Darüber hinaus sollte der Riechstoffspender auch ein müheloses Entsorgen des Riechstoff-bzw. Verdampfungsrückstandes ermöglichen.

Die Lösung der vorerwähnten Aufgaben besteht in einem Riechstoff, dessen Fondnote gegenüber den anderen Riechstoffkomponenten das höchste Wirkstoffquantum besitzt. Des weiteren besteht die Lösung darin, daß bei einem Reeichstoffspender der eingangs erwähnten Art erfindungsgemäß im Bereich einer Heizvorrichtung ein Riechstoffhalter und/oder Dosierer angeordnet ist, der der Heizvorrichtung je Benutzung eine auf das Wärmeangebot abgestimmte sowie ausreichende und für den Benutzer verträgliche Riechstoffmenge zuführt. Da sich so die Riechstoffdosis auf das Wärmeangebot der Heizvorrichtung, beispielsweise eines elektrisch, katalytisch oder gasbeheizten Brenners abstimmen läßt, ergibt sich nicht nur eine hohe Ausbeute ohne Überdosierung, sondern auch ein rückstandsarmes Verdampfen.

Die Erfindung umfaßt drei Komponenten: den Riechstoff, den Riechstoffträger und den Riechstoffbehälter.

Eine Voraussetzung zu der Problemlösung liegt in der Anwendung der ätherischen Öle unter Vermittlung eines Riechstoffträgers. Ohne Speicherung eines flüssigen Riechstoffs in einem Behälter oder beispielsweise einem festen Riechstoffträger wäre eine einfache Dosierung nicht möglich. Die gegenüber der bisherigen zweiteiligen Produktstruktur (riechstoff und riechstoffflasche) erfindungsgemäße dreiteilige Produktstruktur erlaubt eine jederzeitige Riechstoff-bzw. Aromaanwendung oder -therapie ohne jeden besonderen Aufwand in homöopathischen, in jedem Falle aber nichttoxischen und auch geruchssympathischen Dosen.

Die Rezeptur eines Riechstoffes für einen festen Riechstoffträger muß nicht nur dem speziellen aromatherapeutischen Ziel, sondern auch in besonderer Weise dem Umstand Rechnung tragen, daß die einzelnen Riechstoff-Komponenten verschiedene Flüchtigkeiten (Siedepunkte) besitzen. Entsprechend ihrer unterschiedlichen Flüchtigkeiten ist zwischen Kopfnoten, Herznoten und Fondnoten zu unterscheiden.

Da bislang nur flüssige Riechstoffkompositionen bekannt sind, kam ihrer Wirkung nach sofortiger Entnahme aus der Flasche und damit den leicht flüchtigen Stoffen, d.h. den Kopfnoten, die entscheidende Bedeutung zu. Diese leicht flüchtigen Stoffe sind jedoch bei der Nutzung über einen festen Riechstoffträger nur von dienender Bedeutung, weil sie sich zumeist nicht dauerhaft speichern lassen.

Die erfindungsgemäße Riechstoffkomposition geht daher nicht von den Kopfnoten, sondern von den Fondnoten aus.

Demgemäß sind solche Riechstoffe geruchsprägend, die den schwerflüchtigen Fondnoten zuzurechnen sind. Ihr Haftvermögen in einem Träger läßt sich erfindungsgemäß durch hochsiedende Lösungsmittel beispielsweise Benzylbenzoat verstärken.

Soweit einzelne Stoffe unter spezieller therapeutischer Zielsetzung flüchtiger wirken sollen, können diese gleichzeitig an verwandte Fondnoten gebunden und von niedrigsiedenden Lösungsmitteln mobilisiert werden.

So erlaubt ein erfindungsgemäßer Riechstoff den Genuß von Tabakaroma und Nikotin ohne die gesundheitsschädigenden Wirkungen von Kondensatablagerungen aus dem Tabakrauch. Dieser Riechstoff besteht aus einer Komposition von Nikotinderivaten mit anregenden und kreislaufharmonisierenden Aromen, bei der der Nikotinanteil ohne Wirkunsverlust gegenüber dem Rauchtabak minimal ist, ohne dem Raucher ein Entzugserlebnis aufzubürden. Schließlich wirkt der gefäßverengenden Wirkung des Nikotins die kontinuierliche Aufnahme in homöopathischen Dosen entgegen.

Der Trägerstoff besteht erfindungsgemäß aus Alpha-Zellstoff-Komprimat, geschäumtem Kunststoff (Polypropylene, Polyamide) oder pyrogener Kieselsäure als bindendem Trägerstoff.

Diese Riechstoffträger werden den Forderungen gerecht, die sich aus dem Wirkungsziel der Riechstoffrezeptur ergibt; sie sind geruchs-und geschmacksneutral; gehen keine Verbin dung mit den Riechstoffen ein, die diese in ihrer Wirkung verändert und besitzen eine große Speicherkapazität; sie sind resistent gegenüber den aggressiven ätherischen Ölen. Dabei hängt die Speicherkapazität von zwei Faktoren ab, dem Porenvolumen einerseits und der Anzahl der Poren je Kubikzentimeter d.h. der Feinporigkeit. Besonders geeignet sind Trägerstoffe mit einer Speicherkapazität von 40 bis 70% ihres Volumens.

Die Riechstoffträger sind so formbar, daß sie sich leicht dosieren, beispielsweise pelletieren lassen. Weiterhin sind die Riechstoffträger abriebfest, und zwar sowohl gegenüber der ständigen Bewegung einzelner Trägerteile untereinander, als auch gegenüber den Wandungen eines Riechstoffbehälters. Schließlich ist die Oberfläche der Riechstoffträger elastisch genug, um bei der Berührung mit der Innenwandung des Riechstoffbehälters keine Geräusche zu verursachen.

Um ein problemloses Entsorgen insbesondere im Falle eines unerwünschten, beispielsweise klebrigen Verdampfungsrückstandes zu gewährleisten, kann der Riechstoffbehälter mit einem Entsorgungsbehälter beispielsweise in einander gegenüberliegender Anordnung verbunden sein. Der Entsorgungsbehälter kann dabei, wenn sein Fassungsvermögen nicht der Lebensdauer des Riechstoffspenders entspricht, auswechselbar befestigt sein; er ist im Hinblick auf eine gute Reststoffsicherung vorzugsweise mit einer Einwegöffnung versehen und kann mit dem Riechstoffbehälter, gegebenenfalls aber auch mit einem Brennstoffbehälter eine Baugruppe bilden, um ein gleichzeitiges Nachfüllen und Entsorgen durch bloßes Auswechseln der Baugruppe zu ermöglichen.

Des weiteren kann bei dem erfindungsgemäßen Riechstoffspender oberhalb der Heizvorrichtung ein verschließbarer, beispielsweise kaminartiger Riechkanal angeordnet sein, um eine bessere Konvektion zu erreichen. Der Riechkanal kann verschlossen sein, um einen Riechstoff oder Reststoffaustritt zu verhindern und eine Verunreinigung des Spenders zu vermeiden.

Um auf einfache Weise den Riechstoff vom Riechstoff-bzw. Vorratsbehälter in den Bereich der Heizvorrichtung und von dort den Verdampfungsrückstand zum Entsorgungsbehälter bringen zu können, sind der Riechstoff-und der Entsorgungsbehälter vorzugsweise beiderseits der Heizvorrichtung angeordnet. Dabei kann die Auslaßöffnung des riechstoffbehälters über einen Riechstoffhalter bzw. eine Riechstoffführung im Ein-

flußbereich der Heizvorrichtung mit der Einlaßöffnung des Entsorgungsbehälters verbunden und beispielsweise an der Auslaßöffnung des Riechstoffbehälters ein bis in den Riechstoffhalter bzw. die Riechstoffführung oder bis in den Bereich der Einlaßöffnung des Entsorgungsbehälters wirksamer Dosierstößel oder -schieber angeordnet sein. Diese Zuodnung empfiehlt sich insbesondere, wenn der Riechstoffbehälter unter dem Einfluß der Schwerkraft geschieht. Im Falle eines pastösen oder flüssigen Riechstoffs empfiehlt sich jedoch ein federbelasteter Kolben, der den Riechstoff unter Druck hält.

Besonders bedienungsfreundlich und zudem sicher gegen einen Fehlgebrauch ist ein Riechstoffbehälter, bei dem der Riechstoff an einem Trägerband angeordnet ist sowie der Riechstoffbehälter und der Entsorgungsbehälter eine Kassette mit mindestens einer Spule und einem Steg bilden. In diesem Fall läuft das riechstoffdotierte Trägerband, beispielsweise aus zwei miteinander verschweißten Metallfolien, zwischen denen Riechstoffpillen oder Mikrokapseln eingeschlossen sind, von einer Vorratsspule ab auf eine Entsorgungsspule. Kapseln eignen sich insbesondere für Riechstoff, die der Gefahr biochemischer Umsetzungen und/oder eines Austrocknens unterliegen, wie das bei der Verwendung von Tabakstaub der Fall sein kann. Alternativ könnten jedoch auch einzelne Portionen von einem Träger-bzw. Kapselband abgetrennt werden, das von einer Vorratsspule abläuft.

Das Trägerband kann aus einer mit dem Riechstoff imprägnierten Tabakfolie bestehen, wie sie auch beim Herstellen von Tabakerzeugnissen verwendet wird. Derartige Tabakfolien bestehen aus einem mit einer Aufschlämmung von Tabakstaub getränkten Trägervlies beispielsweise aus Zellulose. In diesem Falle dient der Tabak als Riechstoffträger sowie als Riechstoff, da die Aromastoffe des Tabaks mit in die Duftnote eingehen. Das Trägerband kann auch ausschließlich aus Riechstoff gebildet sein. Um die Reißfestigkeit zu erhöhen und gleichzeitig eine Vergleichmäßigung des Wärmeeinbringens zu erreichen, kann das Trägerband mindestens mit einer Metallfolie, beispielsweise auf seiner der Heizvorrichtung zugekehrten Seite mit vorzugsweise einer Aluminiumfolie, kaschiert sein. Andererseits kann das Trägerband aber auch zur Ganze aus einer mit dem Riechstoff versehenen, bei spielsweise mit den Riechstoff enthaltenen Mikrokapseln oder mit einer Aufschlämmung aus Tabakstaub und Riechstoffe beschichteten Aluminiumfolie bestehen. Eine derartige Folie wirkt in der Spule als Verdampfungsschutz und bewirkt zudem eine rasche und gleichmäßige Wärmeverteilung.

Das Trägerband kann teilweise perforiert sein, um dessen Vorschub mit Hilfe einer Stachelwalze zu bewirken. Bei einer Anordnung des Riechstoffs in den Löchern eines perforierten Trägerbandes können die in die Perforation eingreifenden Stacheln der Walze zum Reinigen des Trägerbands nach der Abgabe des Riechstoffs im Bereich der Heizvorrichtung dienen. Die Löcher des Trägerbands können alsdann in einem speziellen Riechstoffbehälter erneut mit einem beispielsweise pastösen Riechstoff gefüllt und als umlaufendes Trägerband erneut der Heizvorrichtung zugeführt werden.

Die Heizvorrichtung besteht vorzugsweise aus einem an einen beispielsweise mittels Solarzellen aufladbaren Akku angeschlossenen Heizdraht, einer Heizrolle oder -platte, über die das Trägerband geführt ist. Andererseits läßt sich ein metallisches Trägerband aber auch zonenweise direkt unter Strom setzen und auf diese Weise örtlich auf die erforderliche Temperatur für das Verdampfen des Riechstoffs bringen.

Andererseits kann aber zur geordneten Zuführung von pastösem oder stückigem Riechstoff auch ein Drehteller oder eine mit Taschen versehen Portionierwalze die Riechstoffdosierung übernehmen, wenn diese einerseits im Bereich der Auslaßöffnung eines Riechstoffbehälters und andererseits im Bereich der Heizvorrichtung angeordnet sind. Auf diese Wei se läßt sich mit einfachen Mitteln, beispielsweise im Verlauf einer 180°-Drehung eine Riechstoffportion vom Riechstoffbehälter zur Heizvorrichtung bringen.

Die Anordnung eines Riechstoffbehälters mit einem Tropfendosierer oberhalb der Heizvorrichtung, beispielsweise einer Heizmulde, vorzugsweise mit etwa W-förmigem Querschnitt, bei unterhalb des Muldenzentrums angeordnetem Brenner empfiehlt sich vor allem bei der Verwendung fluider, beispielsweise flüssiger Riechstoffe. Dabei kann der Riechstoffbehälter den Riechkanal konzentrisch umgeben. die Heizmulde dient als Riechstoffhalter und erlaubt ein indirektes Beheizen des Riechstoffs, um auf diese Weise ein örliches Überhitzen des riechstoffs ebenso wie unerwünschte Reaktionen mit einer Brennerflamme, aber auch ein Verschmutzen der Heizvorrichtung durch den Riechstoff oder dessen Rückstände zu vermeiden.

Schließlich kann der riechstoffspender je nach Verträglichkeit des Riechstoffs bzw. der Verdampfungsrückstände auch mit einem Sicherheitsverschluß versehen sein. Dieser Sicherheitsverschluß befindet sich vorzugsweise an einem Austauschteil aus Riechstoffbehälter, Entsorgungsbehälter und Riechkanal, um ein gekapseltes Entsorgen zu ermöglichen, aber auch die Langzeitstabilität des Riechstoffs zu gewährleisten, sofern dieser nicht selbst gekapselt ist.

Ein Riechstoffspender ohne besonderen Riechstoff behälter ist erfindungsgemäß mit einem im Einflußbereich einer Heizvorrichtung angeordneten Riechstoffhalter versehen. und weist eine Erwärmungskammer, eine Brennkammer, eine Zünderkammer und eine Brennstoffkammer auf. Mit hilfe der Heiz vorrichtung gelingt es bei dem vorzugsweise stabförmigen Riechstoffspender in der Größe etwa eines Taschenfeuerzeuges unter dem Einfluß von Wärme den Riechstoff mit gleichbleibender Intensität auch über einen längeren Zeitraum freizusetzen. Dabei ist die Heizvorrichtung vorzugsweise unterhalb eines siebförmigen Riechstoffhalters angeordnet, um den an einen Träger gebundenen Riechstoff durch Strahlung und Konvektion erwärmen zu können.

Der Zünder läßt sich auf einfache Weise betätigen, wenn mindestens ein Durchlaß in einer Gehäusekuppe mit einem vorzugsweise eine Schwalbenschwanzführung aufweisenden Schieber verschlossen und dieser Schieber mit einem Auslöser des Zünders verbunden ist. Gleichzeitig kann der Schieber auch mit einem Tankventil verbunden sein, so daß mit der Öffnungsbewegung des Schiebers gleichzeitig die Zündung betätigt und das Tankventil geöffnet wird.

Insgesamt ergibt sich somit ein Riechstoffspender für eine diskontinuierliche Benutzung, der den sich aus der Beschaffenheit des jeweiligen Riechstoffs ergebenden Erfordernissen - im Einzelfall auch auf unterschiedliche Weise - Rechnung trägt, da beispielsweise nicht in allen Fällen eine sichere Entsorgung erforderlich ist. Der dem jeweiligen Riechstoff angepaßte Dosierer gewährleistet zudem ein stets gleiches und gleichbleibendes Wirkstoffquantum. Da er den Riechstoffbehälter abschließt, sind Frische und Qualität des Riechstoffs gewahrt.

Der Riechstoffspender läßt sich ohne Schwierigkeiten in einer Hand halten und mit einem Daumen wie ein Feuerzeug betätigen. Dabei wird die Heizvorrichtung sofort eingeschaltet, wenn sich der Riechstoffdurchlaß öffnet und bleibt so lange eingeschaltet, bis der Schieber den Riechstoffdurchlaß wieder verschließt.

Um die Riechstoff-Moleküle jederzeit und überall blitzschnell freizugeben, ist der Spender kleinformatig; er läßt sich schnell öffnen und -schließen. Eine bequeme Handhabung erlaubt den Riechstoffdurchlaß einhändig und ohne optische Kontrolle zu bedienen.

Die Erfindung wird nachfolgend anhand von in der Zeichnung dargestellten Ausführungsbeispielen des näheren erläutert, die weitere Einzelheiten der Erfindung erkennen lassen. In der Zeichnung zeigen:

Fig. 1 einen axialen Längsschnitt durch einen stabförmigen Reichstoffspender für einen fluiden Riechstoff mit kreisförmigem Querschnitt;

Fig. 2 einen Querschnitt nach der Linie A-A in Fig. 1,

Fig. 3 einen Querschnitt nach der Linie B-B in Fig. 1,

Fig. 4 einen Querschnitt nach der Linie C-C in Fig. 1,

Fig. 5 einen axialen Längsschnitt durch einen stabförmig-rechteckigen Riechstoffspender für Riechstoffpads,

Fig. 6 einen Längsschnitt nach der Linie A-A in Fig. 5,

Fig. 7 einen Querschnitt nach der Linie B-B in Fig. 5,

Fig. 8 einen Querschnitt nach der Linie C-C in Fig. 5,

Fig. 9 einen Riechstoffspender für pastösen Riechstoff,

Fig. 10 einen Schnitt nach der Linie A-A in Fig. 3,

Fig. 11 einen Schnitt nach der Linie B-B in Fig. 3,

Fig. 12 einen Schnitt nach der Linie C-C in Fig. 3,

Fig. 13 einen dem Ausführungsbeispiel der Fig. 9 ähnlichen Riechstoffspender, jedoch für einen pillenförmigen Riechstoff,

Fig. 14 einen Schnitt nach der Linie A-A in Fig. 13,

Fig. 15 einen Schnitt nach der Linie B-B in Fig. 13,

Fig. 16 einen Schnitt nach der Linie C-C in Fig. 13,

Fig. 17 einen Längsschnitt durch einen Riechstoffspender mit einem Riechband in einer Kassette,

Fig. 18 eine Draufsicht auf den Riechstoffspender der Fig. 17,

Fig. 19 das Antriebssystem für die Spule der Kassette des Riechstoffspenders nach Fig. 17,

Fig. 20 einen Querschnitt nach der Linie A-A in Fig. 19,

Fig. 21 einen als Kapselband ausgebildeten Riechstoffträger,

Fig. 22 einen dosenförmigen Riechstoffspender,

Fig. 23 einen Längsschnitt durch einen Riechstoffspender mit einem Dosieröffnungen aufweisenden Trägerband,

Fig. 24 einen vertikalen Längsschnitt nach der Linie A-A in Fig. 23,

Fig. 25 eine Draufsicht auf den Riechstoffspender nach Fig. 23 und

Fig. 26 den Bandantrieb des Riechstoffspenders nach Fig. 23 mit einer Draufsicht auf das Trägerband.

Fig. 27 einen Riechstoffspender ohne Riechstoffbehälter,

Fig. 28 die Seitenansicht eines dosenförmigen Riechstoffspenders,

Fig. 29 einen als Nachfüllpackung geeigneten Riechstoffspender,

Fig. 30 einen Teil des Riechstoffspenders der Fig. 29.

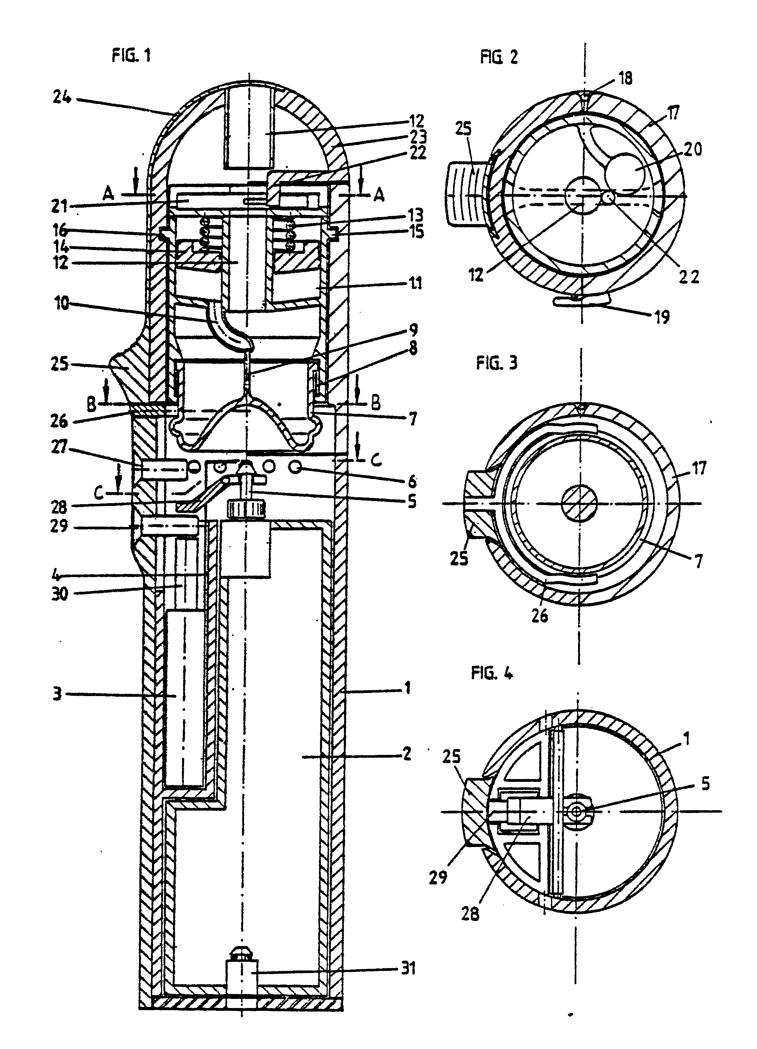

Der Riechstoffspender besteht in seiner einfachsten Form gemäß Fig. 1 aus einem Gehäuse 1 mit einem Gastank 2 und einem piezoelektrischen Zünder 3, dessen Zündfahne 4 ober halb eines Brennerventils 5 endet. Das Brennerventil 5 ist im Bereich von Luftöffnungen 6 so angeordnet, daß sich die Brennerflamme etwa im Zentrum einer W-förmigen Heizmulde 7 befindet, die in einer Gleitführung 8 axial verschiebbar im mittleren Teil des Gehäuses 1 gelagert ist. Die Heizmulde 7 trägt in ihrem Zentrum eine Ventilnadel 9, die in die Ventilöffnung einer Dosiertülle 10 eingreift. die Einlaßöffnung der Tülle 10 befindet sich im Boden eines ringförmigen Riechstofftanks 11, durch dessen Zentrum sich ein zweiteiliger Riechkanal 12 erstreckt. Der Riechkanal 12 ist von einem ringförmigen, unter dem Druck einer Feder 13 stehenden Kolben 14 umgeben. Der Riechstoffbehälter 11 bildet mit der Heizmulde eine Baueinheit; diese ist mittels eines Bundes 15 in einer Gehäusenut 16 gehalten und läßt sich nach der Erschöpfung des Riechstoffs durch Öffnen einer Türe 17 radial aus dem Gehäuse 1 herausnehmen. Die Türe 17 ist über ein Scharnier 18 am Gehäuse 1 angelegt und weist einen Schnappverschluß 19 auf.

Un ein lagegerechtes Einsetzen des Riechstoffbehälters 11 zu ermöglichen und gleichzeitig einen schwenkbar gelagerten Riechkanaldeckel 20 zum umweltschonenden Verschließen des Reichkanals seitwärts zu schwenken, weist der Riechstofftank 11 einen Querschlitz 21 auf, in den eine gehäusefeste Führungsnase 22 eingreift.

Nach oben läuft das Gehäuse 1 in eine Kuppel 23 mit der Mündung des Riechkanals 12 aus, die mit Hilfe einer in einer Schwalbenschwanzführung verschiebbaren Jalousie 24 verschlossen ist. Die Jalousie 24 ist Bestandteil eines Schiebers 25 mit drei Mitnehmern 26, 27, 29. Der gabelförmige Mitnehmer 26 dient zum axialen Verschieben der Heizmul de 7 und damit auch der Ventilnadel 9, der Mitnehmer 27 zum Betätigen eines Ventilhebels 28 für das Brennerventil 5 und der Mitnehmer 29 zum Betätigen des Zünderauslösers 30.

Der Gastank 2 weist in seinem boden ein Nachfüllventil 31 auf, während der Riechstofftank 11 mit der Heizmulde 7 als durch den Schwenkdeckel 20 verschlossenes Wegwerfventil ausgebildet ist, um ein umweltschonendes Entsorgen zu ermöglichen.

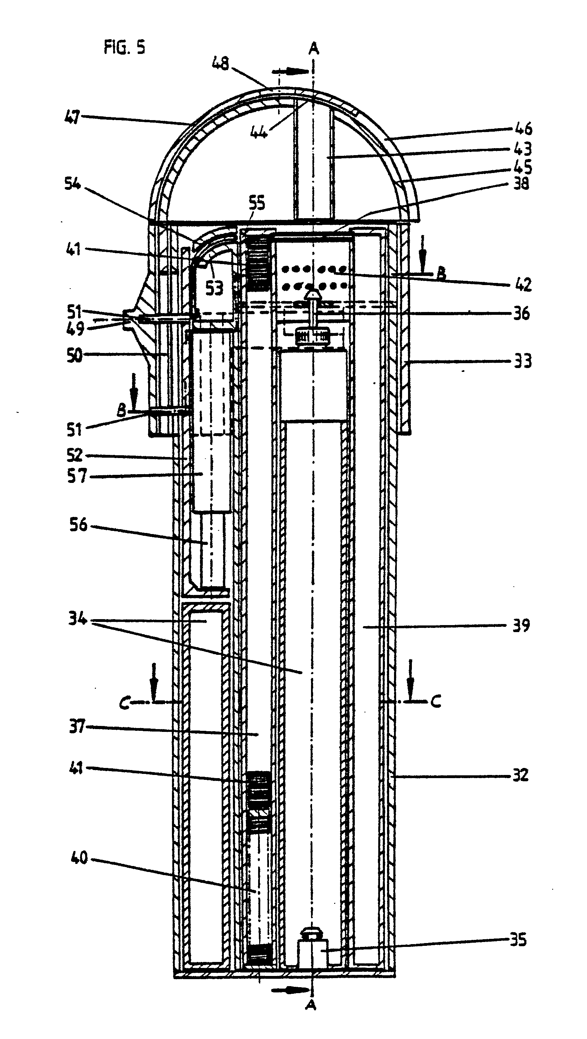

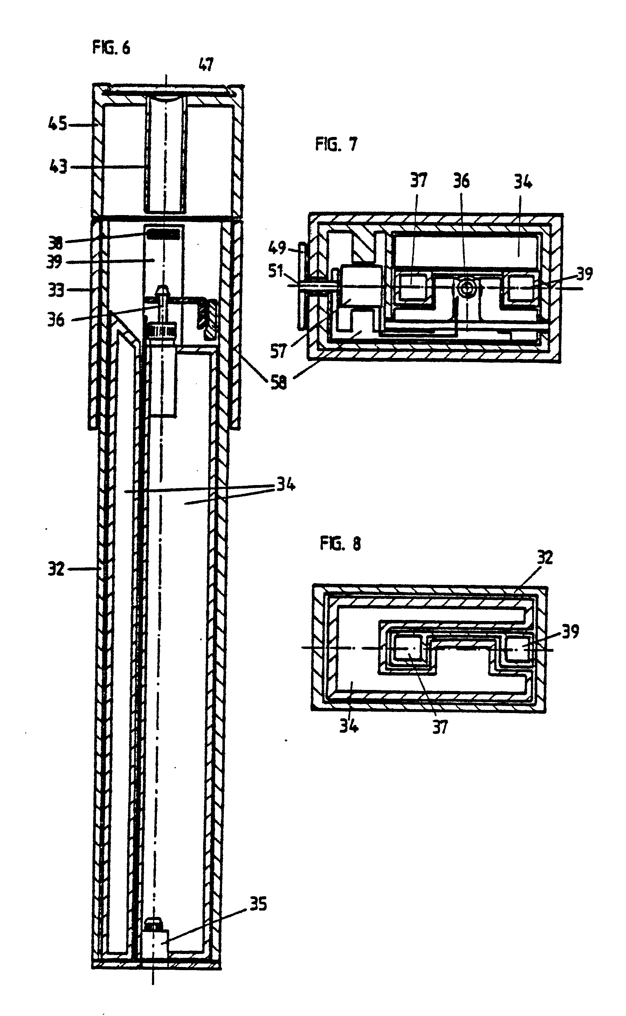

Der Riechstoffspender nach den Fig. 5 bis 8 ist zur Verwendung eines festen Riechstoffs bzw. Riechstoffträgers in form einzelner Pads bestimmt;

er besteht aus einem im Querschnitt rechteckigen Gehäuse 32 mit einer aufgesteckten, jedoch gehäusefesten Kappe 33. In dem Gehäuse befindet sich ein Gastank 34 mit einem Nachfüllventil 35 und einem Brennerventil 36 sowie ein Reichstoffschacht 37, der an seinem oberen Ende über eine Riechstofführung 38 mit einem Entsorgungsschacht 39 verbunden ist. Am Fuße des Riechstoffschachts 37 befindet sich eine Vorschubfeder 40, die die vor ihr leigenden Riechstoffpads 41 zum Kopfende des Schachts 37 drückt. Im Bereich des Brennerventils 36 befinden sich Luftöffnungen 42, und oberhalb des Brennerventils 36 endet ein Riechstoffkanal 43, dessen Austrittsöffnung 44 in einer Kuppe 45 liegt, die schwenkbar mit dem Spendergehäuse 32 verbunden ist. Die Spenderkuppe 45 besitzt eine Schwalbenschwanzführung 46, in der sich eine Jalousie 47 mit einer Riechöffnung 48 verschieben läßt.

Zum Öffnen wird die Jalousie 47 mit Hilfe eines Schiebers 49 so lange verschoben, bis die Riechkanalöffnung 44 und die Riechöffnung 48 zur Deckung gekommen sind.

Mit dem Schieber 49 ist über zwei durch ein Gehäusefenster 50 ragende Mitnehmer 51 ein L-förmiger Hebel 52 zum Betätigen eines in einer Führung 53 verschiebbaren Stößels 54 verbunden. Die Führung 53 endet fluchtend vor einer Öffnung 55 des vorratsschachts 37, die ihrerseits mit der Riechstofführung 38 fluchtet. Der L-förmige Hebel endet vor dem Stößel 56 eines piezoelektrischen Zünders 57 und betätigt über einen weiteren L-förmigen Hebel 58 das Brennerventil 36.

Bei einer Benutzung des Riechstoffspenders der Fig. 5 braucht lediglich der Schieber 49 nach oben geschoben zu werden, bis die Riechkanalöffnung 44 und die Riechöffnung 48 in der Jamousie 47 zur Deckung gekommen sind. Während des Öffnens werden mit einer geringfügigen Verzögerung das Brennerventil 36 geöffnet und der piezoelektrische Zünder 57 betätigt und gelangt gleichzeitig mit Hilfe des Stößels 54 das zu oberst liegende Pad in die Führung 38, die in dem Bereich zwischen Riechkanal 43 und dem Brennerventil 36 perforiert ist, um den Wärmeübergang für das Freisetzen des Riechstoffs zu verbessern. Beim Einschieben des in den Vorratsschacht 37 zuoberst liegenden Pads mit Hilfe des Stößels 54 wird gleichzeitig der Rest des zuvor benutzten Pads aus der Führung 38 in den Entsorgungsschacht 39 gedrückt.

Wenn der vorrat in dem Schacht 37 erschöpft ist, läßt sich die im wesentlichen U-förmige Baueinheit aus dem Vorrats schacht 37, der Padführung 38 und dem Entsorgungsschacht 39 mühelos nach oben aus dem Gehäuse 32 herausziehen und auswechseln, nachdem die Gehäusekuppe 45 mit dem Riechkanal 43 und der Jalousie 47 nach außen geschwenkt worden ist.

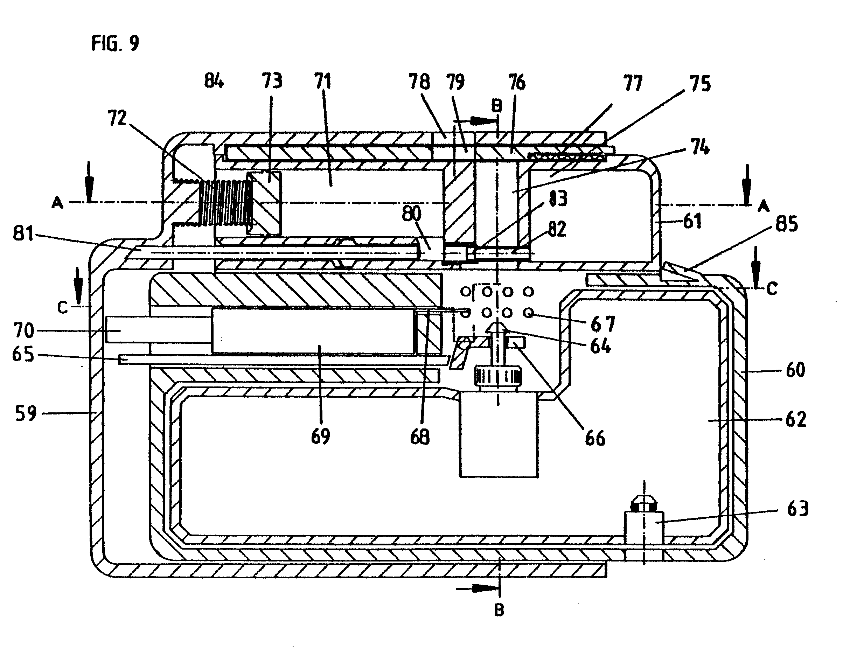

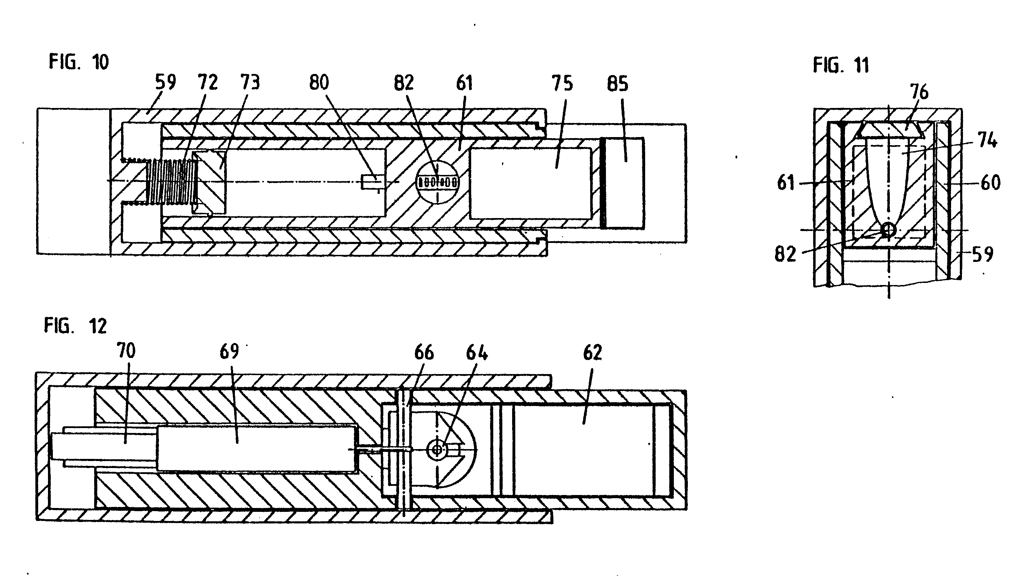

Bei dem Riechstoffspender für pastösen Riechstoff der Fig. 9 bis 12 ist in einem einseitig offenen kastenförmigen Schieber 59 ein Gehäuse 60 sowie ein Austauschteil 61 mit einem insgesamt der Innenkante des Schiebers 59 entsprechenden Querschnitt gelagert. In dem Gehäuse befindet sich ein Gastank 62 mit einem Nachfüllventil 63 und einem Brennerventil 64, das mittels eines das Gehäuse 60 überragenden Stößels 65 über einen verschwenkbar gelagerten Kniehebel 66 betätigt wird. Im Bereich des Brennerventils 64 sind Luftöffnungen 67 und die Zündfahne 68 eines piezoelektrischen Zünders 69 angeordnet, dessen Betätigungsstößel 70 das Gehäuse 60 ebenfalls überragt.

Die Austauscheinheit 61 besteht aus einer Riechstoffkammer 71 mit einem unter dem Druck einer sich am Schieber 59 abstützenden Feder 72 stehenden Kolben 73 sowie einem Riechkanal 74 und einer Entsorgungskammer 75. Der Riechkanal 74 ist im Ruhezustand stets durch einen Sicherheitsschieber 76 verschlossen, der mit Hilfe einer Feder 77 in seiner Schließstellung gehalten wird. Der Sicherheitsschieber 76 besitzt einen mit einer Riechöffnung 78 im kastenförmigen Schieber 60 fluchtenden Durchlaß 79.

Von der riechstoffkammer 71 führt eine Dosierkammer 80 mit einem im Schieber 59 gelagerten Dosierstößel 81 zu einer die Riechstoffkammer mit der Entsorgungskammer 75 verbinden den, mit Öffnungen versehenen Riechstoffführung 82. In der Riechstoffführung 82 ist eine elastische Ventilscheibe, beispielsweise ein Silikonventil 83 angeordnet, deren Durchlaß sich automatisch öffnet, wenn der Dosierschieber 80 eine sich aus dem Dosierkammervolumen ergebende Portion Riechstoffpaste in die Riechstoffführung 82 drückt.

Dies geschieht durch eine Relativbewegung zwischen dem kastenförmigen Schieber 59 und dem Gehäuse 60, während derer der schieberfeste Dosierstößel 81 in die Riechstoffführung 82 gelangt; gleichzeitig werden mit einer gewissen zeitlichen Verzögerung der Zünderstößel 70 und alsdann der Stößel 65 betätigt, so daß an der Zündfahne 68 ein Zündfunken entsteht und das Brennerventil 64 geöffnet wird. Gleichzeitig wird auch der Sicherheitsschieber 76 mit Hilfe eines Mitnehmers 84 gegen den Druck der Feder 77 verschoben, bis der Riechkanal 74, der Durchlaß 79 und die Riechöffnung 78 fluchten, so daß der unter dem Einfluß der Brennerflamme verdampfende Riechstoff austreten kann.

Wenn der Riechstoffvorrat erschöpft ist, läßt sich das Austauschteil 61 nach dem Herabdrücken einer Klipp-Arretierung 85 am Gehäuse 60 ohne weiteres aus dem kastenförmigen Schieber 59 herausziehen und durch ein neues Austauschteil ersetzen. Der Sicherheitsschieber 76 verbleibt dabei in seiner in Fig. 9 dargestellten Lage und verhindert so ein Austreten der in der Entsorgungskammer 75 befindlichen Reststoffe.

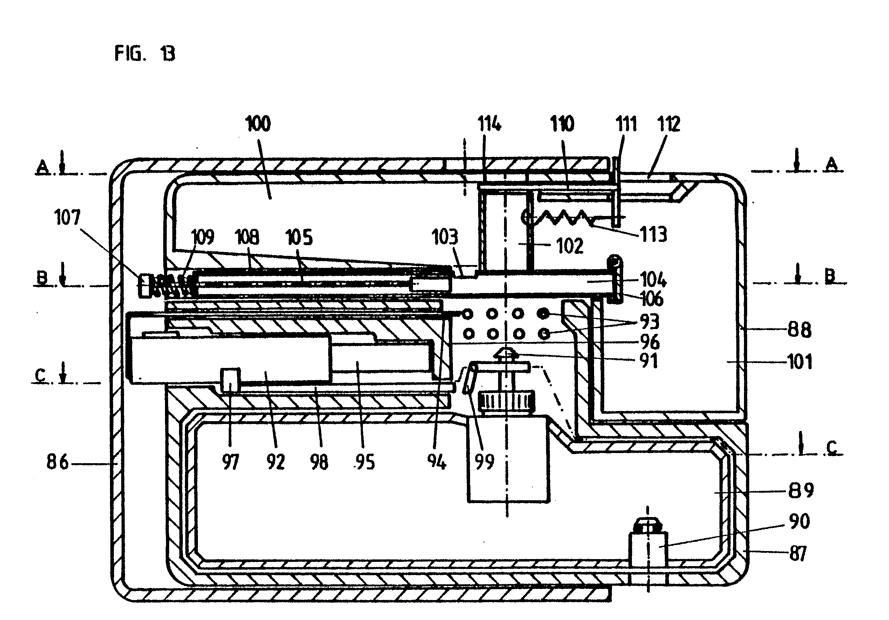

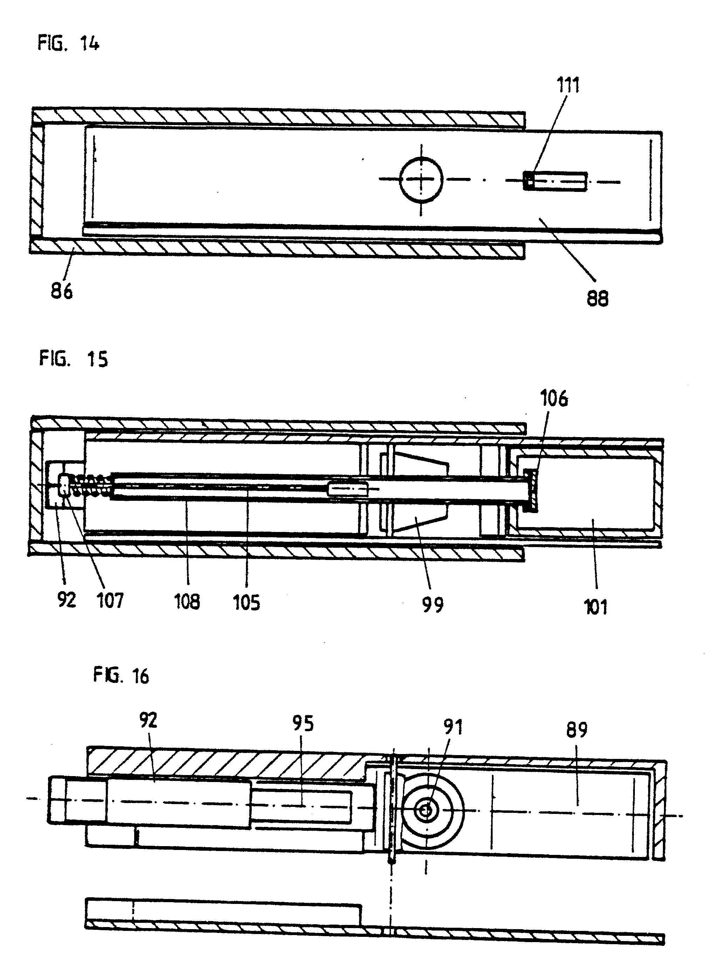

Bei dem Riechstoffspender nach den Fig. 13 bis 16 sind ähnlich wie bei dem Riechstoffspender der Fig. 9 bis 12 ein einseitig offener kastenförmiger Schieber 86 einerseits sowie ein Tankgehäuse 87 und - als Austauschteil - ein Riechstoffgehäuse 88 andererseits verschiebbar ineinander geschachtelt. Das Tankgehäuse 87 enthält einen Gastank 89 mit einem Nachfüllventil 90 und einem Brennerventil 91 sowie einem verschiebbar gelagerten piezoelektrischen Zünder 92 mit einer im Bereich von Luftöffnungen 93 und des Brennerventils 91 endenden Zündfahne 94. Der Betätigungsstößel 95 des das Gehäuse 87 in Richtung des Schiebers 86 überragenden Zünders 92 endet an einer Gehäusenase 96. Über einen Mitnehmer 97 ist der Zünder 92 mit einem Stößel 98 verbunden, der über einen verschwenkbar gelagerten Kniehebel 99 das Brennerventil 91 betätigt.

Das Riechstoffgehäuse 88 besteht aus einer Vorratskammer 100 für Riechstofftabletten oder Kapseln und einer Entsorgungskammer 101 sowie einem Riechkanal 102. Von der vorratskammer 100 führt ein Durchlaß 103 zu einer Riechstofführung 104 mit einem Dosierschieber 105 und einer Entsorgungsklappe 106. Die Riechstofführung 104 ist im Bereich des Brennerventils 91 bzw. des Riechkanals 102 perforiert. Der Dosierschieber 105 weist an seiner Schubstange eine Kopfscheibe 107 auf, zwischen der und einem Schiebergehäuse 108 sich eine Druckfeder 109 erstreckt.

Oberhalb des Riechkanals 102 befindet sich ein Verschlußschieber 110 mit einem Stift 111 in einer Gehäuseöffnung 112, dessen eines Ende über eine Zugfeder 113 mit dem Riechkanal 102 verbunden ist und dessen anderes Ende vor der Stirnkante des kastenförmigen Schiebers 86 liegt.

Beim Verschieben des kastenförmigen Schiebers 86 sowie der Gehäuse 88, 89 gegeneinander werden der Zünder 92, der Dosierschieber 105 und der Stift 111 in ihre jeweilige Arbeitsstellung sowie eine Riechöffnung 114 im Schieber 86 zur Deckung mit dem Riechkanal 102 gebracht.

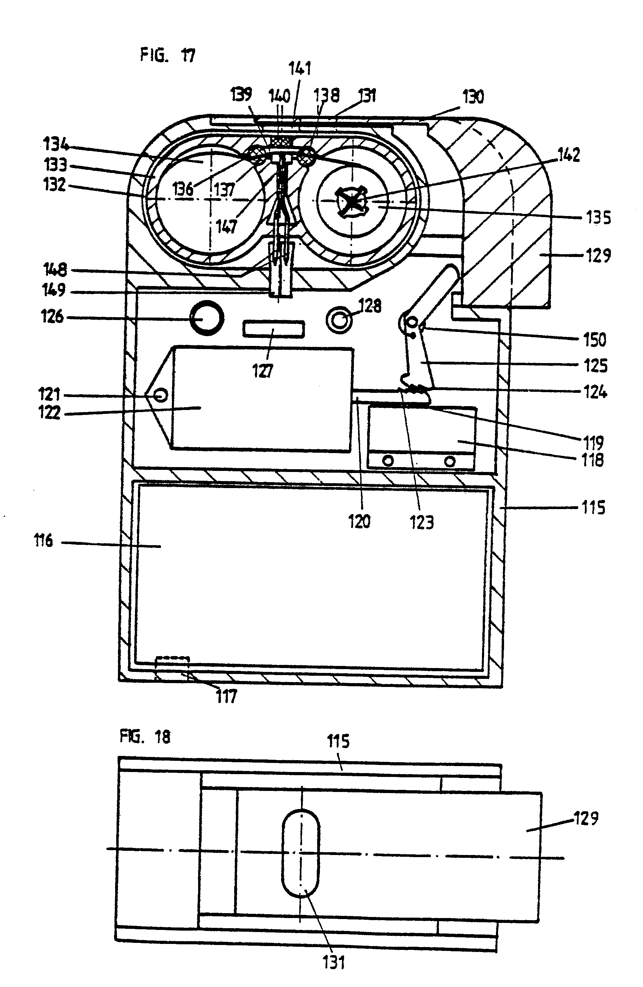

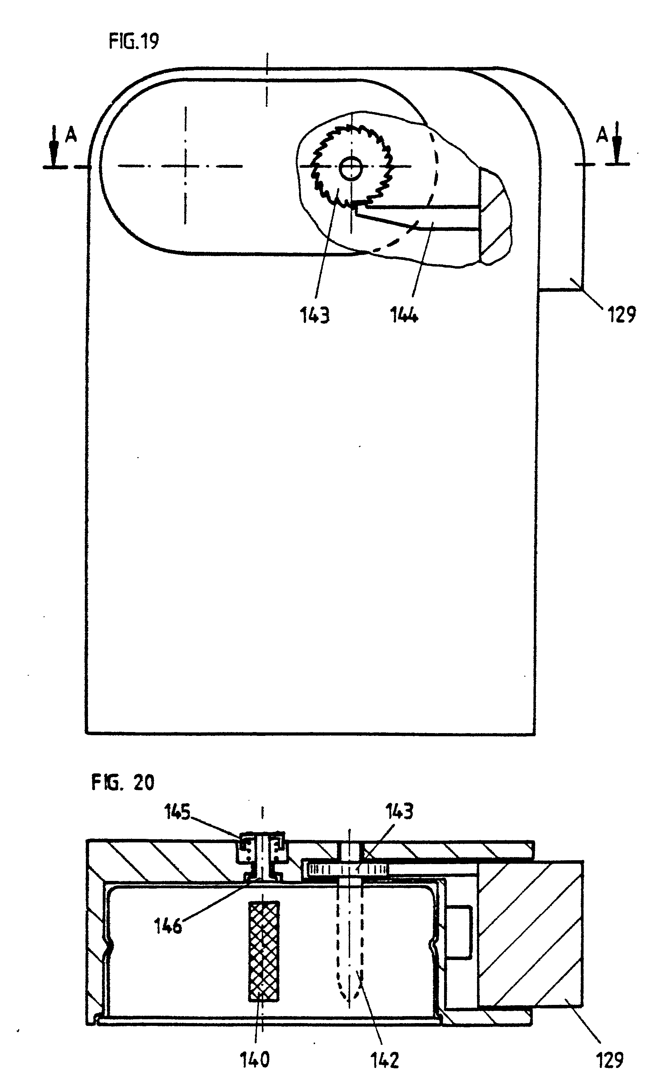

Der Riechstoffspender nach den Fig. 17 bis 20 besteht aus einem einheitlichen Gehäuse 115, in dessen unterem Teil ein aufladbarer Akku 116 mit einem Aufladekontakt 117 angeordnet ist. Oberhalb des Akkus befindet sich ein Mikroschalter 118 mit einem federbelasteten Schaltknopf 119, über dem der Schaltfinger 120 eines in einem Drehpunkt 121 verschwenkbar gelagerten pneumatischen oder hydraulischen Zeitgebers 122 liegt. Der Schaltfinger 120 besitzt ein Sägezahnprofil 123, in das das Sägezahnprofil 124 eines drehbar gelagerten Kniehebels 125 eingreift.

Oberhalb des Zeitgebers 122 sind ein Ladekontrollknopf 126, ein Anschlag 127 und eine Ladekontrollampe 128 angeordnet.

Das obere Ende des Kniehebels 125 befindet sich vor einem im Gehäuse 115 geführten Auslöser 129, in dessen Schließlasche 130 sich eine Riechöffnung 131 befindet. Im oberen Teil des Gehäuses 115 ist in einer Kassettenkammer 132 eine Kassette 133 mit einem Riechstoffwickel 134 und einer Entsorgungsspule 135 angeordnet. Zwischen den beiden Spulen 134, 135 verläuft ein mit dem Riechstoff versehenes Trägerband 136 durch eine beiderseits mit Dichtungen 137, 138, beispielsweise Lippendichtungen, versehenen Riechstoff-bzw. Bandführung 139. In der Bandführung befindet sich ein gasdurchlässiges Gitter 140 unterhalb einer Gehäuseöffnung 141.

Die Entsorgungsspule 135 sitzt auf einem Dorn 142, in dessen Klinkenrad 143 ein mit dem Auslöser 129 verbundener Klinkenhebel 144 eingreift.

Die Kassette 133 liegt bündig in dem Gehäuse 115 und läßt sich mittels eines federbelasteten Knopfes 145 und eines Zapfens 146 seitlich aus dem Gehäuse drücken und gegen eine neue Kassette auswechseln.

Unterhalb des Gitters 140 befindet sich ein Heizdraht 147, dessen Messerkontakte 148 in nach außen offene Schlitze zweier Kontaktstücke 149 eingreifen, die über nicht dargestellte Leitungen und den Mikroschalter 118 mit dem Akku 116 verbunden sind. Beim Eindrücken des Auslösers 129 bewegt sich das untere Ende des Kniehebels 125 mit dem Schaltfinger 120 entgegen der Bewegungsrichtung des Auslösers, bis der Schaltfinger 120 frei kommt und der Zeitgeber 122 unter dem Einfluß des federbelasteten Schaltknopfs 119 bis zu seinem Anschlag 127 nach oben schwenkt. Dabei wird der Schaltknopf 119 freigegeben, so daß der Heizdraht 147 mit Strom versorgt wird, bis der Auslöser 129 schließlich unter dem Einfluß einer auf den Kniehebel 125 wirkenden Drehfeder 150 ebenso wie der Zeitgeber 122 in die Ausgangslage zurückkehrt.

Das Trägerband 136 kann aus Tabakfolie beispielsweise einem mit einer Aufschlämmung von Tabakstaub getränkten Cellulosevlies bestehen und mit dem Riechstoff imprägniert sein; seine Reißfestigkeit liegt so hoch, daß sie das Verdampfen des Riechstoffs bei erhöhter Temperatur übersteht. Um das zu gewährleisten kann das Trägerband auch eine Randverstärkung aufweisen. Der Riechstoff läßt sich jedoch auch se parat, beispielsweise über eine als Schwamm ausgebildete Dichtung 137 zuführen oder auch aufspritzen. Andererseits kann das Trägerband 136 auch als Kapselband ausgebildet sein, dessen Kapseln 151 sich zwischen zwei Metallfolien 152, 153 befinden und ein Gemisch aus Tabak, Aroma, Salz, Wasser und Nikotin enthalten. An die Stelle einer Kapsel können auch mehrere Mikrokapseln treten. Solche Mikrokapseln eignen sich auch zum Beschichten eines Trägerbandes beispielsweise aus Aluminium.

Um ein Herausziehen des Trägerbandes mit einem spitzen Gegenstand zu verhindern, kann an der Auslaßöffnung der Vorratsspule über dem Trägerband ein sich senkrecht zum Trägerband erstreckendes Trennmesser angeordnet sein. An die Stelle des Trennmessers können auch einzelne, das Trägerband blockierende und je nach Belastung auch trennende Stacheln treten.

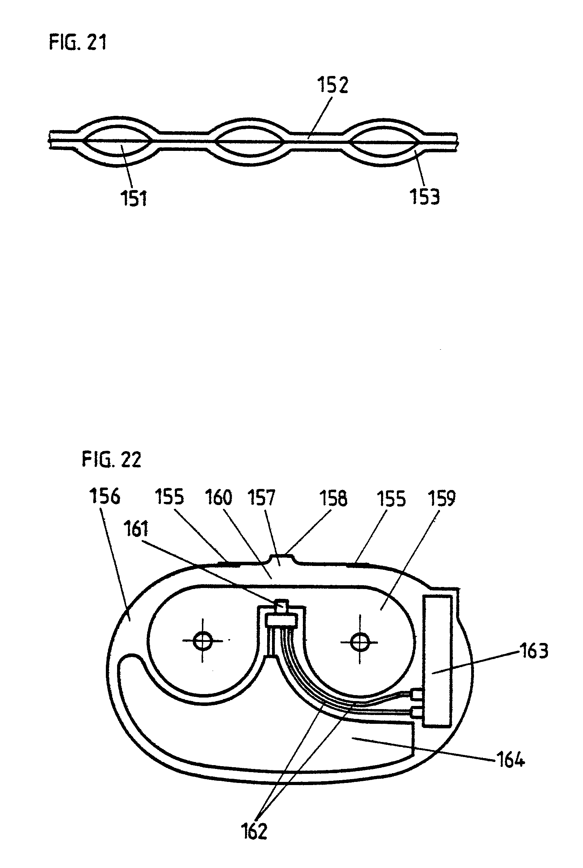

Der aus zwei über Scharniere 155 miteinander verbundenen Schalen 156 bestehende Riechstoffspender der Fig. 22 weist an einer Nase 157 eine Riechöffnung 158 auf und enthält eine Kassette 159. Der Steg 160 der Kassette 159 ist als Riechstoffhalter bzw. -führung ausgebildet; er befindet sich unterhalb der Riechöffnung 158 und oberhalb eines katalytischen Brenners 161, der über Leitungen 162 mit einem Zünder 163 verbunden ist und aus einem Gastank 164 gespeist wird.

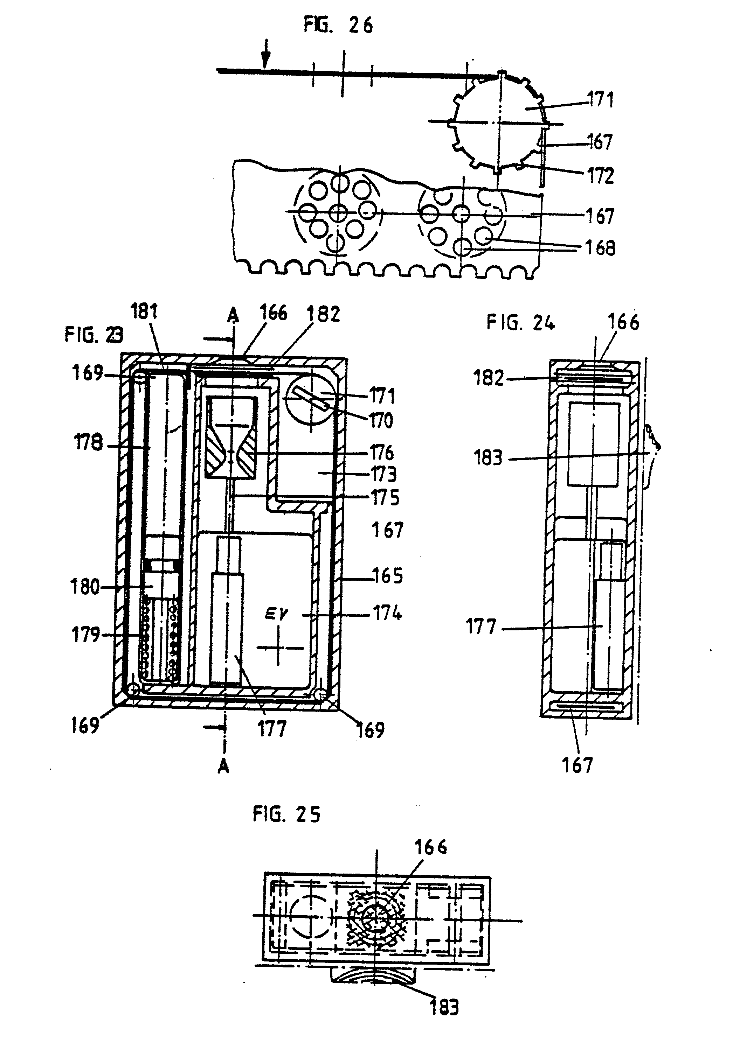

Der Riechstoffspender nach den Fig. 23 bis 26 weist in einem Gehäuse 165 mit einer Riechöffnung 166 ein Dosierband 167 mit gruppenweise angordneten Dosieröffnungen 168 auf.

Das Dosierband 167 ist über Rollen 169 sowie eine mit einem Knebel 170 versehene Stachelwalze 171 geführt, deren Stacheln 172 in die Löcher 168 eingreifen. Unterhalb der Stachelwalze 171 befindet sich eine Entsorgungskammer 173.

Das Gehäuse 165 enthält des weiteren einen Gastank 174, von dem eine Leitung 175 zu einem unterhalb der Riechöffnung 166 angeordneten katalytischen Brenner 176 führt. In diesen Brenner ragt die nicht dargestellte Zündfahne eines Zünders 177.

Weiterhin enthält das Gehäuse 165 eine Riechstoffpatrone 178 mit einem unter dem Einfluß einer Druckfeder 179 stehenden Kolben 180, vor dem sich eine Riechstoffpaste befindet, die von dem Kolbendruck durch eine Öffnung 181 in der Stirnseite der Patrone 178 in die vor der Öffnung liegende Lochgruppe eingedrückt wird. Bei einer Drehung der Stachelwalze 171 mittels des Knebels 170 gelangt diese Lochgruppe in eine perforierte Dosierbandführung 182 zwischen der Riechöffnung 166 und dem Brenner 176 sowie nach einer weiteren Drehung in den Einflußbereich der Stachelwalze 171, deren Stacheln 172 in die Löcher 168 eindringen und dort vorhandene Riechstoffreste entfernen; diese sammeln sich in der Entsorgungskammer 173.

Der Zünder 177 wird durch einen Schieber 183 betätigt, mit dessen Hilfe auch die Leitung 175 geöffnet wird. Dies geschieht über eine nicht dargestellte Hebelmechanik.

Die Riechstoffpatrone 178, die drei Rollen 169 und die Stachelwalze sind auf einem gemeinsamen (nicht dargestellten) Träger, beispielsweise einer Platte, angeordnet und bilden ein Austauschteil. Das Gehäuse 165 ist mit einer nicht dargestellten Türe versehen, nach deren Öffnen sich der Träger aus dem Gehäuse herausnehmen und die Entsorgungskammer 173 reinigen läßt.

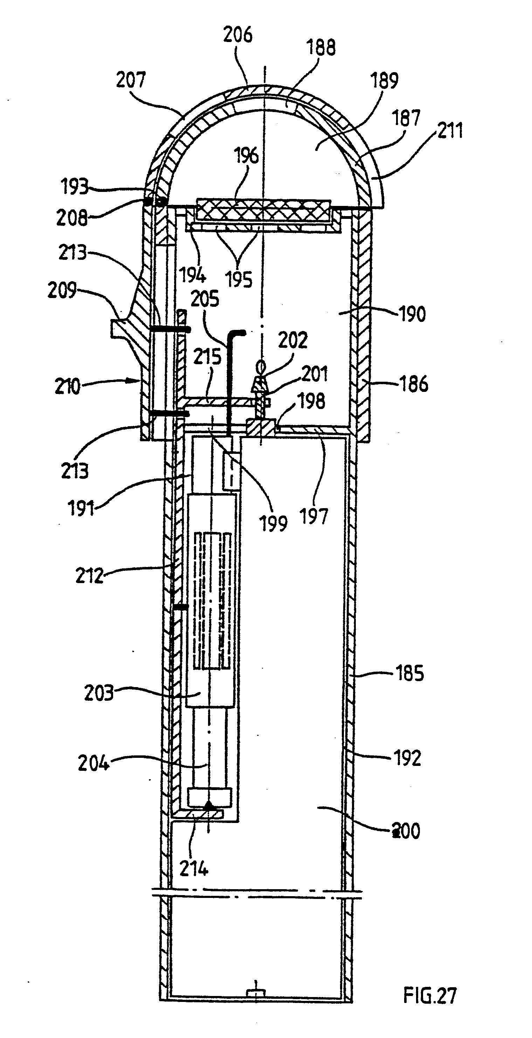

Der Riechstoffbehälter nach Fig. 27 besteht aus einem im Querschnitt rechteckigen Gehäuse 185 mit einer aufgesteckten Kappe 186, in deren Kuppe 187 sich ein Riechstoffdurchlaß 188 befindet. Der Riechstoffbehälter ist in vier Kammern, d.h. in eine Erwärmungskammer 189, eine Brennkammer 190, eine Zünderkammer 191 und eine Brennstoffkammer 192 gegliedert.

Die Erwärmungskammer 189 ist nach oben hin durch die über ein Gelenk 193 mit der Steckkappe 186 verbundene Kuppe 187 und nach unten hin durch einen Riechstoffhalter 194 in Form eines Öffnungen 195 aufweisenden Siebbodens begrenzt. Auf dem Siebboden 194 ruht ein Riechstoffträger 196 mit inkorporiertem Riechstoff. Die unterhalb des Siebbodens 194 befindliche Brennkammer 190 ist durch einen Zwischenboden 197 mit zwei Öffnungen 198, 199 begrenzt. Unterhalb des Zwischenbodens 197 befindet sich ein eingesteckter Brennstofftank 200 mit einem Brenner 201. Der Brenner 201 durchragt die Zwischenbodenöffnung 198 und besitzt ein Ventil 202.

Neben dem Brennstofftank 200 ist ein piezoelektrischer Zünder 203 mit einem Zünderstößel 204 und einer den Zündfunken leitenden Fahne 205 angeordnet. Die Fahne 205 durchragt die Zwischenbodenöffnung 199 und endet im Bereich des Brennerventils 202.

Die Behälterkuppe 187 überdeckt eine Jalousie 206 mit einem Durchlaß 207, die über ein Gelenk 208 mit einem eine Griffnase 209 aufweisenden Schieber 210 verbunden und in einer Schwalbenschwanzführung 211 geführt ist. Mit dem Schieber 201 ist eine im Gehäuseinnern angeordnete Betätigungslasche 212 über Stifte 213 verbunden. Diese Betätigungslasche 212 weist zwei Ausleger 214, 215 auf. Der Ausleger 214 liegt an der Stirnseite des Zünderstößels 204 an, während der Ausleger 215 den Brenner 201 mit Spiel umschließt und unterhalb des Brennerventils 202 liegt.

Wird der Schieber 210 aus der in Fig. 27 dargestellten Lage nach oben geschoben, dann bewegt sich die Jalousie 206 in der Schwalbenschwanzführung 211, bis die beiden Durchlässe 188, 207 zur Deckung kommen. Gleichzeitig bewegt sich die Betätigungslasche 212 nach oben, betätigt mit ihrem Ausleger 214 über den Stößel 204 den Zünder 203 und öffnet mit Hilfe ihres Auslegers 215 das Brennerventil 202, so daß die Brennerflamme gezündet wird.

Die aus der Verbrennung resultierende Warmluft gelangt durch die Brennkammer 190 und die Siebbodenöffnungen 195 in die Erwärmungskammer 189. Dabei umspült sie den Riechstoffträger 196, der in Abhängigkeit von dem Wärmeangebot den Riechstoff freigibt. Dieser gelangt durch die beiden sich mehr oder minder deckenden Durchlässe 188, 207 nach außen.

Die Öffnungsweite wird dabei durch die Lage der Jalousie 206 auf der Kuppe 187 bestimmt. Dieser Lage entspricht jeweils eine bestimmte Öffnungsstellung des Ventils 202; denn das Ventil wird durch den Laschenausleger 215 in dem Maße geöffnet, wie sich die beiden Durchlässe 188, 207 über decken. Erst wenn sich beide Durchlässe voll überdecken, ist auch das Ventil 202 voll geöffnet und entfaltet der Brenner 201 seine volle Leistung.

Auf diese Weise ermöglicht der erfindungsgemäße Riechstoffbehälter eine dosierte Riechstoffabgabe, die sofort beendet ist, wenn der Durchlaß 188 durch die Jalousie 206 wieder verschlossen und gleichzwitig auch das Ventil 202 wieder geschlossen ist. Je nachdem, in welcher Höhe der Ausleger 215 an der Lasche 212 angeordnet ist, kann das Ventil 202 auch vor dem Schließen des Durchlasses 188 die Brennstoffzufuhr unterbrechen, um einen Druck-und Temperaturstau in der Erwärmungskammer 189 und der Brennkammer 190 zu vermeiden.

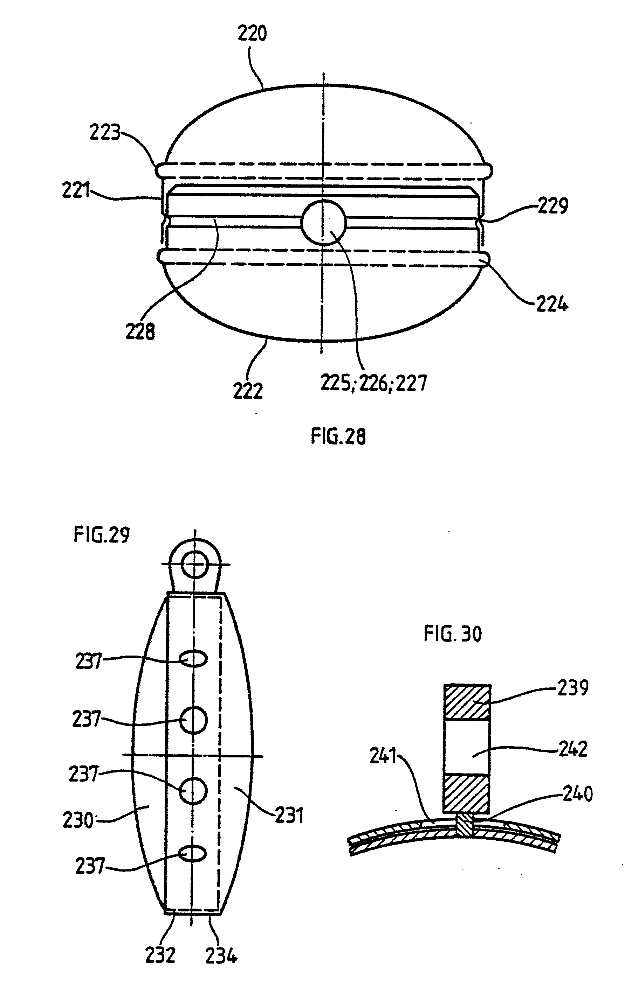

Der erfindungsgemäße Reichstoffspender gemäß Fig. 28 besteht aus einer Kappe 220, die mit ihrem schürzenartigen Rand 221 eine Dosenschale 222 übergreift. Die Kappe 220 und die Dosenschale 222 sind mit Hilfe eines Bördelrands 223, 224 versteift und weisen jeweils drei auf gleicher Höhe angeordnete Austrittsöffnungen 225, 226, 227 auf. Diese Austrittsöffnungen sind aus Gründen einer besseren Ventilation um jeweils 120° gegeneinander versetzt und liegen sämtlich im Bereich einer umlaufenden Rille 228 der Dosenschale 222, in die eine umlaufende Sicke 229 der Kappenschürze 221 eingreift. Infolge der Elastizität der Kappenschürze 221 schnappt die Sicke 229 beim Zusammenstecken der beiden Dosenteile 220, 222 leicht in die Umfangsrille 228 der Dosenschale 220 ein.

Bei dem Ausführungsbeispiel der Fig. 29, 30 besteht die Reichstoffdose aus zwei gleichartigen Teilen, d.h. aus einer Dosenschale 230 und einem Dosendeckel 231 jeweils mit einem zylinderförmigen Randabschnitt 232, 234. Die Randabschnitte 232, 234 sind konzentrisch zueinander angeordnet und weisen in gleicher Höhe Austrittsöffnungen 237 auf, die einander gruppenweise gegenüberliegen, um eine gewisse Querventilation zu ermöglichen.

An dem zylindrischen Randabschnitt 232 der Dosenschale 230 befindet sich eine Schiebeöse 239, deren Fuß 240 sich durch eine Öffnung 241 im außenliegenden Randabschnitt 234 des Dosendeckels 231 ersteckt; sie weist eine Öffnung 242 zum Befestigen einer nicht dargestellten Haltekette auf.

**Ansprüche**

1. Riechstoffkomposition, gekennzeichnet durch einen Reichstoff, dessen Fondnote gegenüber den anderen Riechstoffkomponenten das höchste Wirkungsquantum besitzt.

2. Riechstoffkomposition nach Anspruch 1, dadurch gekennzeichnet, daß mindestens eine Riechstoffkomponente durch ein niedrigsiedendes Lösungsmittel mobilisiert ist.

3. Riechstoffkomposition nach Anspruch 1 oder 2, dadurch gekennzeichnet, daß der Riechstoff mindestens ein Nikotinderivat und mindestens ein anregendes und kreislaufmobilisierendes Aroma enthält.

4. Riechstoffkomposition nach einem der Ansprüche 1 bis 3, dadurch gekennzeichnet, daß der Riechstoff an einen Trägerstoff mit einem Porenvolumen von 40 bis 70% gebunden ist.

5. Riechstoffkomposition nach einem der Ansprüche 1 bis 4, dadurch gekennzeichnet, daß der Trägerstoff aus Alpha-Zellstoff-Komprimat, geschäumtem Kunststoff oder pyrogener Kieselsäure besteht.

6. Reichstoffspender für einen Riechstoff nach den Ansprüchen 1 bis 5 mit einem Riechstoffbehälter oder -träger und mindestens einem verschließbaren Durchlaß für den Riechstoff, dadurch gekennzeichnet, daß im Bereich einer Heizvorrichtung (5; 36; 64; 91; 147; 161; 176) ein Riechstoffhalter (194, 195) und/oder ein Riechstoffdosierer (10; 55; 81; 105; 136; 152, 153; 167) angeordnet ist.

7. Riechstoffspender nach Anspruch 6, dadurch gekennzeichnet, daß ein Riechstoffbehälter (11; 37; 71; 100; 134; 178) mit einem Entsorgungsbehälter (7; 39; 75; 101; 135) verbunden ist.

8. Riechstoffspender nach Anspruch 6 oder 7, dadurch gekennzeichnet, daß der Riechstoffbehälter (11; 37; 71; 100; 134) und/oder der Entsorgungsbehälter (7; 39; 75; 101; 135) auswechselbar im Spendergehäuse (1; 32; 60; 87; 115; 156) angeordnet sind.

9. Riechstoffspender nach einem der Ansprüche 6 bis 8, dadurch gekennzeichnet, daß der Riechstoffbehälter (11; 37; 100; 134) und der Entsorgungsbehälter (7; 39; 101; 135) eine auswechselbare Baugruppe (61; 88) bilden.

10. Riechstoffspender nach einem der Ansprüche 6 bis 9, dadurch gekennzeichnet, daß der Entsorgungsbehälter (39; 75; 101; 135) eine Einwegöffnung besitzt.

11. Riechstoffspender nach einem der Ansprüche 6 bis 10, dadurch gekennzeichnet, daß oberhalb der Heizvorrichtung (5; 36; 64; 91; 147; 161) ein verschließbarer Riechkanal (12; 43; 74; 102) angeordnet ist.

12. Riechstoffspender nach einem der Ansprüche 7 bis 11, dadurch gekennzeichnet, daß der Riechstoffbehälter (37; 71; 100; 134; 178) und der Entsorgungsbehälter (39; 75; 101; 135; 173) beiderseits der Heizvorrichtung (36; 147; 161; 176) oder des Reichkanals (74; 102) angeordnet sind.

13. Riechstoffspender nach einem der Ansprüche 7 bis 12, dadurch gekennzeichnet, daß eine Auslaßöffnung des Riechstoffbehälters (37; 71; 100; 134; 178) über eine Riechstoffführung (38; 82; 104; 139; 160; 182) im Einflußbereich der Heizvorrichtung (36; 64; 91; 147; 161; 176) mit einer Einlaßöffnung des Entsorgungsbehälters (39; 75; 101; 135; 173) verbunden ist.

14. Riechstoffspender nach einem der Ansprüche 6 bis 13, dadurch gekennzeichnet, daß der Riechstoff (151) an einem Trägerband (136; 152, 153; 167) angeordnet ist und der Riechstoffbehälter (134) und der Entsorgungsbehälter (135) eine Kassette (133; 159) mit mindestens einer Spule (134; 135) und einem Steg (139; 160) bilden.

15. Riechstoffspender nach einem der Ansprüche 7 bis 14, dadurch gekennzeichnet, daß an der Auslaßöffnung (80; 103) des Riechstoffbehälters (71; 100) ein bis in die Riechstoffführung (82; 104) zum entsorgungsbehälter (75; 101) wirksamer Dosierstößel oder -schieber (81; 105) angeordnet ist.

16. Reichstoffspender nach Anspruch 13 oder 15, dadurch gekennzeichnet, daß die Austrittsöffnung (80; 103) des Riechstoffbehälters (71; 100) höher als die Eintrittsöffnung des Entsorgungsbehälters (75; 101) angeordnet ist.

17. Riechstoffspender nach einem der Ansprüche 6 bis 9, dadurch gekennzeichnet, daß der Riechstoffbehälter (11) oberhalb einer Heizmulde (7) angeordnet und mit einem Tropfendosierer (9, 10) versehen ist.

18. Riechstoffspender nach Anspruch 17, dadurch gekennzeichnet, daß der Riechstoffbehälter (11) den Riechkanal (12) konzentrisch umgibt.

19. Riechstoffspender nach Anspruch 18, dadurch gekennzeichnet, daß die Heizmulde (11) einen W-förmigen Querschnitt besitzt, unterhalb dessen Zentrum sich ein Brenner (5) befindet.

20. Riechstoffspender nach Anspruch 14, dadurch gekennzeichnet, daß das Trägerband (136) aus einer Tabakfolie besteht.

21. Riechstoffspender nach Anspruch 14, dadurch gekennzeichnet, daß das Trägerband (136) aus Riechstoff besteht.

22. Riechstoffspender nach Anspruch 20 oder 21, dadurch gekennzeichnet, daß das Trägerband (136) mindestens mit einer Metallfolie kaschiert ist.

23. Riechstoffspender nach Anspruch 14, dadurch gekennzeichnet, daß das Trägerband aus mindestens einer Metallfolie (152,153) besteht.

24. Riechstoffspender nach einem der Ansprüche 14 und 20 bis 23, dadurch gekennzeichnet, daß das Trägerband (167) perforiert und über eine Stachelwalze (171) geführt ist.

25. Riechstoffspender nach einem der Ansprüche 5 bis 24, dadurch gekennzeichnet, daß die Heizvorrichtung aus einem an einen Akku (116) angeschlossenen Heizdraht (147), einer Heizrolle oder -platte besteht.

26. Riechstoffspender nach einem der Ansprüche 6 bis 25, dadurch gekennzeichnet, daß im Bereich der Auslaßöffnung des Riechstoffbehälters und der Heizvorrichtung ein Drehteller oder eine Portionierwalze angeordnet ist.

27. Riechstoffspender nach einem der Ansprüche 6 bis 26, dadurch gekennzeichnet, daß ein Riechkanal (74; 102) mit einem Sicherheitsverschluß (76; 110) versehen ist.

28. Riechstoffspender nach einem der Ansprüche 6, 11, 25 oder 27, gekennzeichnet durch einen im Einflußbereich der Heizvorrichtung (201) angeordneten perforierten Riechstoffhalter (194, 195), eine Brennkammer (190), eine Zünderkammer (191) und eine Brennstoffkammer (192).

29. Riechstoffspender nach Anspruch 28, dadurch gekennzeichnet, daß die Heizvorrichtung (201) unterhalb des Riechstoffhalters (194, 195) angeordnet ist.

30. Riechstoffspender nach einem der Ansprüche 6 bis 29, gekennzeichnet durch ein stabförmiges Gehäuse (185) und mit einem Durchlaß (188) in einer Gehäusekuppe ·(187) verschließenden Jalousie (206) in einer Schwalbenschwanzführung (211).

31. Riechstoffspender nach einem der Ansprüche 6 bis 30, dadurch gekennzeichnet, daß die Jalousie (206) mit einem Schieber (210) für den Zünder (203) und/oder mit dem Ventil (202) eines im Tankdeckel angeordneten Brenners (201) verbunden ist.

32. Riechstoffspender für eine Riechstoffkomposition nach den Ansprüchen 6 bis 31, gekennzeichnet durch eine Kappe (220) und eine Dosenschale (222) sowie auf gleicher Höhe angeordnete Austrittsöffnungen (225, 226).

33. Riechstoffspender nach Anspruch 32, dadurch gekennzeichnet, daß eine Sicke (229) in einer Schürze (221) der Kappe (220) in eine umlaufende Rille (228) der Dosenschale (222) eingreift.

34. Riechstoffspender nach Anspruch 33, dadurch gekennzeichnet, daß die Kappe (230) und die Dosenschale (222) konzentrisch zueinander angeordnete Randabschnitte (232, 234) aufweisen.

FIG. 1

FIG 2

FIG. 3

FIG. 4

FIG. 5

FIG. 6

FIG. 7

FIG. 8

FIG. 9

0 268 114

FIG. 10

FIG. 11

FIG. 12

FIG. 13

0 268 114

**FIG. 14**

86     111     88

**FIG. 15**

106

92   107    108    105     99     101

**FIG. 16**

92     95     91     89

FIG. 17

FIG. 18

FIG.19

143   144   129

FIG. 20

145   143

146   129

140   142

FIG. 21

152

151

153

FIG. 22

156    155    160    157    158    155    159

161

162

163

164

0 268 114

FIG. 26

171
167
172
167
168

FIG. 23

181    A    166    182
169
178
171
170
176
173
175
167
180
165
179
174
EY
169    169
177
A

FIG. 24    166
182
183
177
167

FIG. 25

166
183

FIG.27

**FIG.28**

**FIG.29**

**FIG. 30**